# EUROPEAN PATENT SPECIFICATION

(11) **EP 3 395 245 B1**
(45) Date of publication and mention of the grant of the patent: **25.03.2026**
(21) Application number: 16879182.0
(22) Date of filing: 16.11.2016
(51) Int. Cl.: G01R 33/563, G01R 33/56, A61B 5/055

(54) **METHOD AND SYSTEM FOR ACQUIRING AN ADDITIONAL IMAGE, FOR IDENTIFYING PERFUSION CHARACTERISTICS, BY USING AN MRA IMAGE**
VERFAHREN UND SYSTEM ZUR AUFNAHME EINES ZUSÄTZLICHEN BILDES ZUR IDENTIFIZIERUNG VON PERFUSIONSEIGENSCHAFTEN UNTER VERWENDUNG EINES MRA-BILDES
PROCÉDÉ ET SYSTÈME D'ACQUISITION D'IMAGE SUPPLÉMENTAIRE, D'IDENTIFICATION DE CARACTÉRISTIQUES DE PERFUSION, À L'AIDE D'UNE IMAGE D'ARM

(30) Priority: 24.12.2015 KR 20150186801
(43) Date of publication of application: 31.10.2018
(73) Proprietor: Gil Medical Center, Incheon 21565 (KR); Gachon University of Industry-Academic Cooperation Foundation, Seongnam-si, Gyeonggi-do 13120 (KR)
(72) Inventor: HAN, Ye Ji, Seoul 07670 (KR); KIM, Eung Yeop, Incheon 21565 (KR); CHUNG, Jun Young, Incheon 21561 (KR); NOH, Young, Incheon 21565 (KR)
(74) Representative: Müller Schupfner & Partner Patent- und Rechtsanwaltspartnerschaft mbB (Hamburg)
(86) International application number: PCT/KR2016/013191
(87) International publication number: WO 2017/111315

(56) References cited:
- JP-A- 2011 167 333
- JP-A- 2012 196 536
- KR-B1- 101 472 709
- US-A- 5 881 728
- US-A1- 2013 281 831
- US-B1- 6 377 835
- BOCK M ET AL: "Separation of arteries and veins in 3D MR angiography using correlation analysis", MAGNETIC RESONANCE IN MEDICINE, JOHN WILEY & SONS, INC, US, vol. 43, no. 3, 1 January 2000 (2000-01-01), pages 481 - 487, XP002302805, ISSN: 0740-3194
- "MRI of the Lung", 28 October 2008, SPRINGER, ISBN: 978-3-540-34619-7, article H. U. KAUCZOR: "3.4 Image Processing", pages: 29, XP055601969
- CHRISTIAN FINK ET AL: "Regional Lung Perfusion: Assessment with Partially Parallel Three-dimensional MR Imaging", RADIOLOGY, vol. 231, no. 1, 1 April 2004 (2004-04-01), pages 175 - 184, XP055601690

## Description

### [Technical Field]

The present invention relates to a method and a system for obtaining at least one additional image, by which perfusion characteristics for a specific area-of-interest of an object may be identified from an MRA image for the object.

### [Background Art]

Computed tomography (CT) or magnetic resonance imaging (MRI) is performed first to exclude the possibility of cerebral hemorrhage when a patient suspected of suffering acute ischemia cerebral infarction is hospitalized. The diagnosis sensitivity of acute ischemia cerebral infarction in diffusion-weighted imaging (DWI) using MRI is much higher than that of CT, and MRI is preferable compared to CT if other conditions are allowed. MRI is performed in the sequence of gradient-recalled echo imaging, DWI, fluid attenuated inversion recovery (FLAIR), MRA (MR angiography), and dynamic susceptibility-weighted perfusion-weighted imaging. A larger number of brain cells may be saved when cerebral infarction is quickly diagnosed and treated. Accordingly, images have to be obtained as quickly as possible, and contrast-enhanced MRA, by which images may be obtained more quickly to shorten the time for diagnosis through MRA, is becoming popular among the above-mentioned imaging techniques. In this case, a contrast medium must be used once more if it has been already used to obtain a perfusion image, much time and costs are required, and the additionally used contrast medium may do harm to the human body of patients (lowering of renal function) having difficulty discharging the contrast medium.

According to the recent study results, because the prognosis may improve when an acute cerebral infarction patient is treated more quickly, blood vessel images for the carotid arteries and brain arteries of a cerebral infarction patient are essentially necessary before carotid artery thrombus removal treatment. The most important factor for selecting a target patient group is a cerebral infarction portion, and evaluation of a collateral circulation using images of blood vessels is also very important. It is known that a temporary carotid artery contrast technology is very precise in evaluating collateral circulation, but the technology is invasive and it takes a long time to obtain images as compared with the noninvasive imaging techniques. In recent years, studies have suggested that evaluation of collateral circulation may be made by using a source image of perfusion as a method for addressing the problem. However, the technology has a problem of blood vessels not being viewable. Although the dynamic contrast-enhanced MRA provides a degree of information during evaluation of collateral circulation, it cannot provide a sectional image, as compared with the collateral circulation evaluation technique using images of perfusion, and thus accuracy decreases. Accordingly, there is a need for development of a technology for evaluating collateral circulation by obtaining a dynamic contrast-enhanced MRA image and using the same to reconstruct a sectional image.
(Patent document 1) Japanese Patent No. 5,325,795 (2013.10.23)
(Patent document 2) Japanese Patent No. 1997-024047 (1997.01.28)
(Patent document 3) Japanese Patent No. 4,266,574 (2009.05.20)

US 6,377,835 B1 is directed to a method for contrast enhanced magnetic resonance angiography (MRA) for producing images wherein arteries and veins are visually separated. The subject under examination is injected with a contrast agent bolus and a number of 3D magnetic resonance angiography data sets are obtained from the examination subject before, during and after arrival of the bolus in the region of interest. A slice from each of the data sets is selected which contains the region of interest, and an average region of interest signal as a function of time is determined and stored as a reference time curve. In the respective selected slices, a signal-time curve is identified, and each signal-time curve is cross-correlated with the reference time curve. The cross-correlation results are used to form a new three-dimensional data set containing arterial and venous correlation maps.

According to one method of postprocessing early arterial phase data is subtracted from peak venous phase images to suppress arterial signal in venograms. However, M. Bock et al. in: "Separation of arteries and veins in 3D MR angiography using correlation analysis", MAGNETIC RESONANCE IN MEDICINE, JOHN WILEY & SONS, INC vol. 43, no. 3, pages 481-487, instead favour a correlation algorithm to postprocess multiphase 3D MRA data sets to allow the separate visualization of the arterial and venous pulmonary vasculature.

U. Kauczor in "MRI of the lung" (Spinger 2008) provides on page 29 under the heading "3.4 Image Processing" different methods of post-processing of images acquired by MRI to achieve a better visualisation of perfusion. These methods cover subtraction of pre-contrast image data from dedicated contrast-enhanced images. However, this approach is explained to have some drawbacks. According to a different more recent method, perfusion parameters are calculated from 3D MR data sets using a specially adapted interpolation algorithm as well as 3D volume rendering.

Christian Fink et al. evaluate in: "Regional Lung Perfusion: Assessment with Partially Parallel Three-dimensional MR Imaging", Radiology, vol. 231, no. 1, pages 175-184 partially parallel three-dimensional (3D) magnetic resonance (MR) imaging for assessment of regional lung perfusion in healthy volunteers and patients suspected of having lung cancer or metastasis.

### [Disclosure]

### [Technical Problem]

The present invention provides a method and a system for obtaining at least one additional image by which perfusion characteristics of an area-of-interest may be recognized from a contrast-enhanced MRA image.

### [Technical Solution]

In accordance with an aspect of the present invention, there is provided a method for obtaining a cross-sectional image for identifying perfusion by using an MRA image as described in claim 1.

The three target image frames include one of a first target image frame corresponding to an artery peak time point, a second target image frame corresponding to a vein peak time point, and a third target image frame corresponding to an intermediate time point between the artery peak time point and the vein peak time point.

The method further includes selecting an arbitrary point of an artery related to the area-of-interest of the object and obtaining a first time-intensity curve for a first voxel in the MRA image corresponding to the selected point (S11), and selecting an arbitrary point of a vein related to the area-of-interest of the object and obtaining a second time-intensity curve for a second voxel in the MRA corresponding to the selected point (S12), wherein both the first is determined based on the first time-intensity curve and both the second and third target images is determined based on the second time-intensity curve.

An image frame corresponding to an artery peak time point that represents a maximum intensity is determined from the first time-intensity curve as the first target image frame in S20.

An image frame corresponding to a vein peak time point that represents a maximum intensity is determined from the second time-intensity curve as the second target image frame in S20.

An image frame corresponding to an intermediate time point between the artery peak time point that represents the maximum intensity in the first time-intensity curve and the vein peak time point that represents the maximum intensity in the second time-intensity curve is determined as the third target image frame in S20.

Any one of the remaining image frames except for the first, second, and third target image frames is determined as the reference image frame in S30.

An image frame that represents a minimum intensity in the first time-intensity curve or an image frame that represents a minimum intensity in the second time-intensity curve is determined as the reference image frame for the first target image frame or for the second target image frame in S30.

The MRA image is obtained through a contrast-enhanced scheme in S10.

The area-of-interest may be a brain of the object.

In accordance with another aspect of the present invention, there is provided a system for obtaining an additional image for identifying perfusion by using an MRA image according to claim 4.

The system may further include a display configured to display the at least one cross-sectional image obtained by the microprocessor.

The three target image frames include one of a first target image frame corresponding to an artery peak time point, a second target image frame corresponding to a vein peak time point, and a third target image frame corresponding to an intermediate time point between the artery peak time point and the vein peak time point.

The microprocessor calculates a first time-intensity graph for a first voxel in the MRA image corresponding to an arbitrary point selected from an artery related to the area-of-interest and a second time-intensity graph for a second voxel in the MRA image corresponding to an arbitrary point selected from a vein related to the area-of-interest, determines the first target image frame based on the first time-intensity graph, determines the second target image frame based on the second time-intensity graph, and determines the third target image frame based on the first and second time-intensity graphs.

The microprocessor determines an image frame corresponding to an artery peak time point that represents a maximum intensity from the first time-intensity curve as the first target image frame.

The microprocessor determines an image frame corresponding to a vein peak time point that represents a maximum intensity from the second time-intensity curve as the second target image frame.

The microprocessor determines an image frame corresponding to an intermediate time point between the artery peak time point that represents the maximum intensity in the first time-intensity curve and the vein peak time point that represents the maximum intensity in the second time-intensity curve as the third target image frame.

The microprocessor determines any one of the remaining image frames except for the first, second, and third target image frames as the reference image frame.

The microprocessor determines an image frame that represents a minimum intensity in the first time-intensity curve or an image frame that represents a minimum intensity in the second time-intensity curve as the reference image frame for the first target image frame or for the second target image frame.

### [Brief Description of Drawings]

Fig. 1 is a flowchart illustrating an embodiment of a method for obtaining a sectional image for identifying perfusion according to the present invention;
Fig. 2 is a block diagram illustrating an embodiment of a system for obtaining a sectional image for identifying perfusion according to the present invention;
Fig. 3 is a view illustrating an example of an MRA image;
Fig. 4 is a view illustrating an example of first and second time-intensity graphs obtained according to the present invention; and
Fig. 5 illustrates views for comparing the present invention and a conventional technology, in which Fig. 5A illustrates an example of perfusion images obtained by the conventional technology and Fig. 5B illustrates an example of cross-sectional images for an area-of-interest obtained according to the present invention.

### [Detailed Description of the Invention]

Hereinafter, exemplary embodiments of the present invention will be described in more detail with reference to the accompanying drawings.

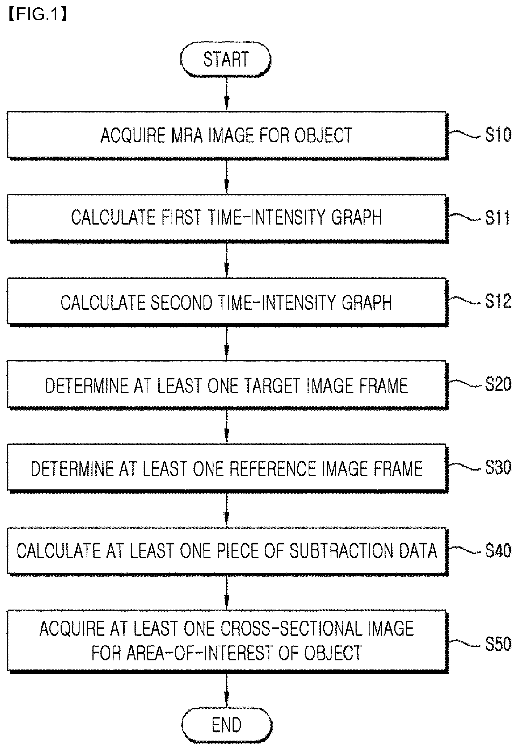

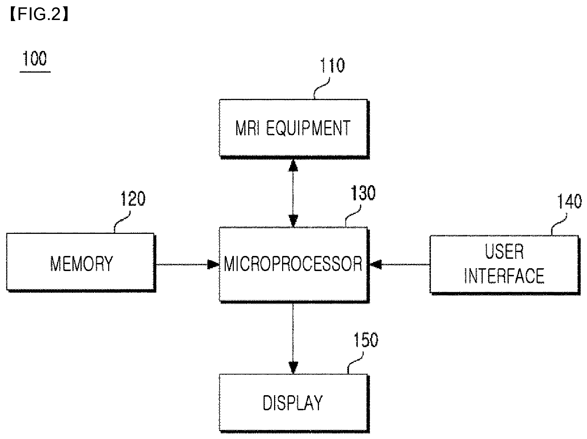

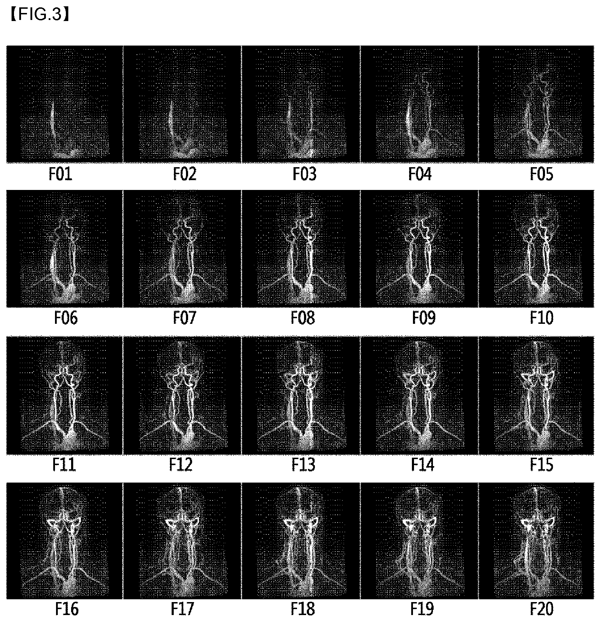

Fig. 1 is a flowchart illustrating an embodiment of a method for obtaining a sectional image for identifying perfusion according to the present invention. Fig. 2 is a block diagram illustrating an embodiment of a system for obtaining a sectional image for identifying perfusion according to the present invention. Fig. 3 is a view illustrating an example of an MRA image.

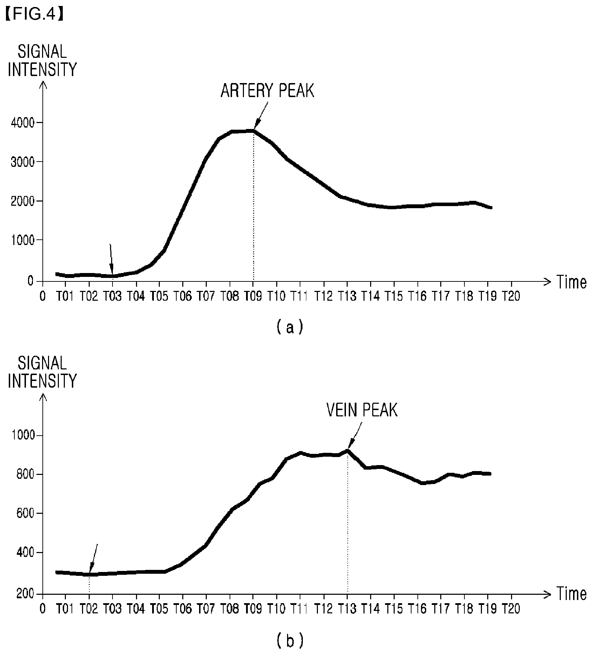

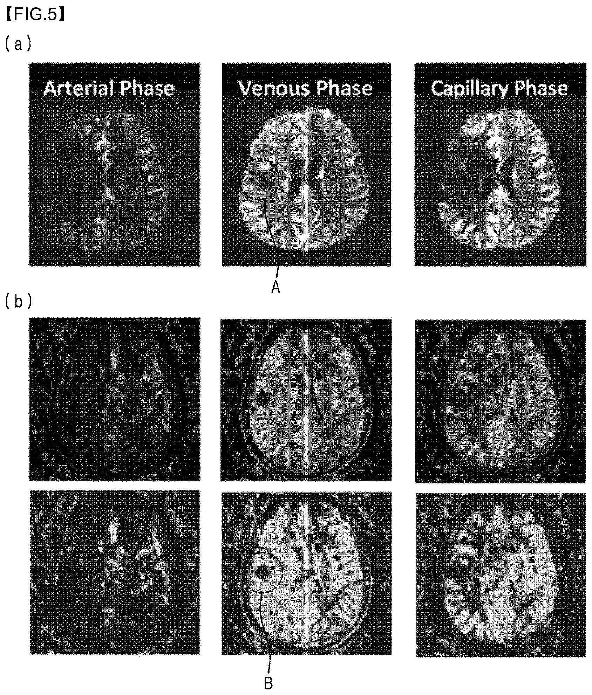

Fig. 4 is a view illustrating an example of first and second time-intensity graphs obtained according to the present invention. Fig. 5 illustrates views for comparing the present invention and a conventional technology, in which Fig. 5A illustrates an example of perfusion images obtained by the conventional technology and Fig. 5B illustrates an example of cross-sectional images for an area-of-interest obtained according to the present invention.

The method according to the embodiment of Fig. 1 and the system according to the system of Fig. 2 are applied to obtain the cross-sectional images of the area-of-interest of the object so as to identify perfusion for the area-of-interest of the object by using an MRA image of the object.

Hereinafter, although it is described that the method of Fig. 1 is realized by the system of Fig. 2, there is no need to limit the system that realizes the method of Fig. 1 to the system of Fig. 2.

The area-of-interest of the object may be a brain of the object. However, there is no need to limit the area-of-interest of the object to a brain, and the present invention may also be applied to obtain additional images for identifying perfusion for another body portion.

The method S100 according to the embodiment of Fig. 1 includes an operation S10 of obtaining an MRA image for an object, an operation S20 of determining at least one target image frame, an operation S30 of determining at least one reference image frame, an operation S40 of calculating at least one piece of subtraction data, and an operation S50 of obtaining at least one cross-sectional image for an area-of-interest.

Additionally, the method S100 of Fig. 1 includes an operation S11 of obtaining a first time-intensity graph, and an operation S12 of obtaining a second time-intensity graph.

The above-mentioned operation will be described in detail as follows.

In operation S10, the MRA image for the object is obtained. The MRA image is an image for observing states of blood vessels of the object, and main arteries and veins that spread out from the heart to the whole body are observation targets. The MRA image may be obtained by using MRI equipment 110 provided in the system 100 of Fig. 2, and the obtained MRA image may be stored in a memory 120 provided in the system 100 of Fig. 2.

As illustrated in Fig. 3, the MRA image includes a plurality of image frames that have been periodically captured for a specific period of time. Although the image frames are illustrated 2-dimensionally in Fig. 3, the data of the image frames is actually 3-dimensional data for the object. Although a total of 20 image frames F01 to F20 are illustrated in Fig. 3, the number of the image frames that constitute the MRA image may be variously changed according to a photographing time and a frame cycle.

The MRA image is obtained through a contrast-enhanced scheme. Here, the contrast-enhanced scheme refers to a scheme of applying a contrast medium to increase the contrast of an image.

In operation S20, three target image frames are determined from a plurality of image frames F01 to F20. The three target image frames include a first target image frame corresponding to an artery peak time point, a second target image frame corresponding to a vein peak time point, and a third target image frame corresponding to an intermediate time point between the artery peak time point and the vein peak time point.

Operations S11 and S12 are performed before operation S20. As mentioned above, operation S11 is an operation of obtaining a first time-intensity graph, and operation S12 is an operation of obtaining a second time-intensity graph.

In detail, in operation S11, if the user selects an arbitrary point of an artery related to an area-of-interest (for example, a brain) of the object by using the MRA image obtained in operation S10, a microprocessor 130 may calculate the first time-intensity graph for a voxel (a first voxel) in the MRA image corresponding to the selected point. The user may select the arbitrary point by using a user interface 140 such as a mouse or a keyboard.

Further, in operation S12, if the user selects an arbitrary point of a vein related to an area-of-interest (for example, a brain) by using the MRA image obtained in operation S10, the microprocessor 130 calculates the second time-intensity graph for a voxel (a second voxel) in the MRA image corresponding to the selected point. The user may select the arbitrary point by using the user interface 140 such as a mouse or a keyboard.

Fig. 4A illustrates an example of the first time-intensity graph, and Fig. 4B illustrates an example of the second time-intensity graph. In the graphs, the X-axis represents time and the Y-axis represents the intensity of a signal. Further, time points T01 to T20, corresponding to the above-mentioned 20 image frames F01 to F20, are displayed in the X-axes of the graphs.

For example, in the first time-intensity graph, a signal intensity displayed at the seventh time point T07 is a signal intensity on the seventh image frame F07 of the first voxel corresponding to the selected artery point. As another example, a signal intensity displayed at the eleventh time point T11 corresponding to the selected vein point is a signal intensity on the eleventh image frame F11 of the second voxel corresponding to the selected vein point. In the first and second time-intensity graphs, the signal intensities change according to time, and this is because the flow rates of the blood passing through blood vessel points corresponding to the first voxel and the second voxel change according to heartrate.

In operation S20, the first target image frame (an image frame corresponding to an artery peak time point) is determined based on the first time-intensity graph, the second target image frame (an image frame corresponding to a vein peak time point) is determined based on the second time-intensity graph, and the third target image frame (an image frame corresponding to an intermediate time point between the artery peak time point and the vein peak time point) is determined based on the first and second time-intensity graphs.

It is assumed that as illustrated in Fig. 4A, the ninth time point T9 corresponds to a maximum intensity and the third time point T03 corresponds to a minimum intensity on the first time-intensity graph, and as illustrated in Fig. 4B, the thirteenth time point T13 corresponds to a maximum intensity and the second time point T02 corresponds to a minimum intensity on the second time-intensity graph.

In this case, the ninth image frame F9 corresponding to the ninth time point T9 may be determined as the first target image frame, the image frame F13 corresponding to the thirteenth time point T13 may be determined as the second target image frame, and the image frame F11 corresponding to an intermediate time point T11 between the artery peak time point T9 and the vein peak time point T13 may be determined as the third target image frame.

In operation S30, at least one reference image frame corresponding to the at least one target image frame is determined from the plurality of image frames F01 to F20. The at least one image frame is determined from the remaining image frames of the plurality of image frames F01 to F20 except for the three target image frames. Individual reference image frames are determined for each of the target image frames, and a reference image frame that is common to the plurality of target image frames may be determined.

For example, when the above-mentioned first, second, and third target image frames F9, F13, and F11 are determined by using the above-mentioned first and second time-intensity graphs in operation S20, individual reference image frames may be determined for the first, second, third target image frames F9, F13, and F11, respectively, and a reference image frame that is common to the first, second, and third target image frames F9, F13, and F11 may be determined in operation S30.

For example, the third image frame F03 corresponding to the third time point T03 that represents a minimum intensity in the first time-intensity graph of Fig. 4A may be determined as a reference image frame for the first target image frame F9, the second image frame F02 corresponding to the second time point T02 that represents a minimum intensity in the second time-intensity graph may be determined as a reference image frame for the second target image frame F13, and any one of the third image frame F03 and the second image frame F02 may be determined as the reference image frame for the third target image frame F11. Alternatively, when a common reference image frame is applied, any one of the third image frame F03 and the second image frame F02 may be determined as a common reference image frame.

When operation S30 is performed based on the above-mentioned first and second time-intensity graphs, it may be automatically performed by the microprocessor 130. In operation S40, three pieces of subtraction data are calculated by subtracting data of a corresponding reference image frame from data of the target image frames. Operation S40 may be performed by the microprocessor 130 of the system 100.

As mentioned above, the image frames correspond to 3-dimensional data. That is, the image frames correspond to a set of a number of voxels arranged 3-dimensionally. Accordingly, the subtraction data obtained in operation S40 also corresponds to 3-dimensional data including a number of voxels.

For example, when the above-mentioned first, second, and third target image frames F9, F13, and F11 are determined in operation S20 and a reference image frame corresponding to the target image frames is determined in operation S30, three pieces of subtraction data (first, second, and third subtraction data) may be obtained by subtracting data of the corresponding reference image frame from the target image frames F9, F13, and F11 in operation S40.

In operation S50, at least one cross-sectional image (additional image) for an area-of-interest (for example, a brain) of the object is obtained based on the subtraction data in operation S40.

Operation S50 may be performed by the microprocessor 130 of the system 100, and the cross-sectional image obtained in operation S50 may be displayed through a display 150 of the system 100.

As mentioned above, the subtraction data obtained in operation S40 also is 3-dimensional data including a number of voxels. Accordingly, in operation S50, one or more cross-sectional images may be obtained for the area-of-interest.

The cross-sectional image obtained in operation S50 may be particularly usefully utilized to identify perfusion of the area-of-interest. Here, identification of the perfusion refers to identification of whether a blood vessel is smoothly supplied through capillary blood vessels to the area-of-interest. In other words, identification of perfusion is identification of whether a necrosis candidate area is present according to insufficient supply of blood to the area-of-interest.

Fig. 5A illustrates an example of perfusion images obtained through CT photographing accompanied by use of a contrast medium according to the conventional technology, and Fig. 5B illustrates an example of additional cross-sectional images obtained in operation S50. In the examples of Figs. 5A and 5B, the area-of-interest is commonly a brain.

In more detail, the cross-sectional images disposed on the left side, the center, and the right side of Fig. 5A are an arterial phase perfusion image, a venous phase perfusion image, and a capillary phase perfusion image obtained through conventional CT photographing.

Meanwhile, the cross-sectional images disposed on the left side, the center, and the right side of Fig. 5B are obtained from the first subtraction data, the second subtraction data, and the third subtraction data obtained in operation S40, and as mentioned above, the first, second, and third subtraction data are calculated based on the first target image frame (corresponding to an artery peak time point), the second target image frame (corresponding to a vein peak time point), and the third target image frame (corresponding to an intermediate between the artery peak and the vein peak).

In the left arterial phase perfusion image and the right capillary phase perfusion image of Fig. 5A, it can be seen that the left brain portion is relatively dark as compared with the right brain portion, and it can be identified that the supply of blood to the left brain portion is not smooth. Further, in the central vein phase perfusion image of Fig. 5A, it can be seen that the left brain portion has a particularly dark area A and the area A is an area that is expected to be a cerebral infarction portion.

Similarly, in the left cross-sectional image and the right cross-sectional image of Fig. 5B, the left brain portion is relatively dark as compared with the right brain portion. Accordingly, it can similarly be identified that the supply of blood to the left brain portion is not smooth by the left and right images of Fig. 5B. Further, in the central cross-sectional image in Fig. 5B, a dark area B appears particularly in the left brain portion. Accordingly, the location of the cerebral infarction portion may similarly be recognized by the left image of Fig. 5B.

Here, it can be seen that the additional images, such as the images of Fig. 5B obtained by applying the present invention, may be sufficiently utilized for recognizing perfusion characteristics of the area-of-interest.

Accordingly, according to the present invention, there is no need to perform additional photographing (for example, CT photographing) accompanied by introduction of a separate contrast medium to recognize perfusion characteristics of the area-of-interest.

Accordingly, a process of diagnosing diseases such as cerebral infarction may become simpler and more inexpensive, and pain, inconvenience, and sideeffects experienced by patients due to introduction of a contrast medium may be remarkably alleviated as the number of introductions of the contract medium decrease as well.

Although the exemplary embodiments of the present invention have been described, an ordinary person in the art to which the present invention pertains will understand that the present invention may be variously corrected and changed without departing from the scope of the present invention claimed in the claims.

## Claims

1. A method for obtaining a cross-sectional image for identifying perfusion by using an MRA image, the method comprising:
obtaining an MRA image including a plurality of 3-dimensional image frames that have been periodically captured for a specific period of time for an object by using MRI equipment (S10), wherein the MRA image is obtained through a contrast-enhanced scheme;
selecting an arbitrary point of an artery related to an area-of-interest of the object and obtaining a first time-intensity curve for a first voxel in the MRA image corresponding to the selected point (S11); and
selecting an arbitrary point of a vein related to the area-of-interest of the object and obtaining a second time-intensity curve for a second voxel in the MRA corresponding to the selected point (S12);
determining three target image frames from the plurality of 3-dimensional image frames (S20) wherein the three target image frames include
a first target image frame corresponding to an artery peak time point,
a second target image frame corresponding to a vein peak time point, and
a third target image frame corresponding to an intermediate time point which is between the artery peak time point and the vein peak time point,
wherein
the first target image frame is determined based on the first time-intensity curve, wherein the first target image frame corresponds to the artery peak time point that represents a maximum intensity in the first time-intensity curve,
the second target image frame is determined based on the second time-intensity curve, wherein the second target image frame corresponds to the vein peak time point that represents a maximum intensity in the second time-intensity curve, and
the third target image frame is determined based on the first time-intensity curve and the second time-intensity curve, wherein the third target image frame corresponds to an intermediate time point between the artery peak time point that represents the maximum intensity in the first time-intensity curve and the vein peak time point that represents the maximum intensity in the second time-intensity curve;
determining three reference image frames corresponding to the first, second and third target image frame from the image frames (S30), wherein any one of the remaining image frames except for the first, second, and third target image frames is determined as reference image frames;
wherein an image frame that represents a minimum intensity in the first time-intensity curve is determined as the reference image frame for the first target image frame; or
wherein an image frame that represents a minimum intensity in the second time-intensity curve is determined as the reference image frame for the second target image frame;
obtaining three pieces of subtraction data by subtracting data of each reference image frame from data of the corresponding three target image frames (S40); and
obtaining the at least one cross-sectional image for the area-of-interest of the object based on the subtraction data (S50).

2. The method of claim 1, wherein
- - the image frame that represents a minimum intensity in the first time-intensity curve, or
- the image frame that represents a minimum intensity in the second time-intensity curve is determined as the reference image frame corresponding to the third target image frame.

3. The method of claim 1, wherein the area-of-interest is a brain of the object.

4. A system for obtaining a cross-sectional image for identifying perfusion by using an MRA image, the system comprising:
MRI equipment configured to provide the MRA image for an object, the MRA image
including a plurality of 3-dimensional image frames that have been periodically captured for a specific period of time;
a user interface (140) configured to allow selection of an arbitrary point of an artery related to an area-of-interest, and configured to allow selection of an arbitrary point of a vein related to an area-of-interest;
a microprocessor configured to
determine three target image frames from the plurality of 3-dimensional image frames, wherein the three target image frames include
a first target image frame corresponding to an artery peak time point,
a second target image frame corresponding to a vein peak time point, and
a third target image frame corresponding to an intermediate time point between the artery peak time point and the vein peak time point,
determine three reference image frames corresponding to the first, second and third target image frame from the image frames;
calculate three pieces of subtraction data by subtracting data of each reference image frame from data of the corresponding three target image frames, and
to obtain the at least one cross-sectional image for an area-of-interest of the object based on the subtraction data,
wherein the microprocessor is configured to
calculate a first time-intensity curve for a first voxel in the MRA image corresponding to an arbitrary point of an artery related to the area-of-interest selected by a user with the user interface (140); and
calculate a second time-intensity curve for a second voxel in the MRA image corresponding to an arbitrary point of a vein related to the area-of-
interest selected by a user with the user interface (140);
determine the first target image frame based on the first time-intensity curve, wherein the first target image frame corresponds to the artery peak time point that represents a maximum intensity in the first time-intensity curve,
determine the second target image frame based on the second time-intensity curve, wherein the second target image frame corresponds to the vein peak time point that represents a maximum intensity in the second time-intensity curve, and
determine the third target image frame based on the first and second time- intensity curve, wherein the third target image frame corresponds to an intermediate time point between the artery peak time point that represents the maximum intensity in the first time-intensity curve and the vein peak time point that represents the maximum intensity in the second time-intensity curve; and
wherein the microprocessor determines any one of the remaining image frames except for the first, second, and third target image frames as the reference image frame, and wherein the microprocessor is configured to determine
an image frame that represents a minimum intensity in the first time-intensity curve as the reference image frame for the first target image frame, or
an image frame that represents a minimum intensity in the second time- intensity curve as the reference image frame for the second target image frame.

5. The system of claim 4, further comprising:
a display configured to display the at least one cross-sectional image obtained by the microprocessor.

6. The system of claim 4, wherein the microprocessor is configured to determine
- the image frame that represents a minimum intensity in the first time-intensity curve, or
- the image frame that represents a minimum intensity in the second time-intensity curve as the reference image frame corresponding to the third target image frame.

## Patentansprüche

1. Verfahren zum Erhalten eines Querschnittsbildes zur Identifizierung von Perfusion unter Verwendung eines MRA-Bildes, wobei das Verfahren umfasst:
Erhalten eines MRA-Bildes, das eine Vielzahl von dreidimensionalen Bildrahmen enthält, die periodisch für eine spezifische Zeitspanne für ein Objekt unter Verwendung einer MRI-Ausrüstung (S10) aufgenommen wurden, wobei das MRA-Bild durch ein kontrastverstärktes Schema erhalten wird;
Auswählen eines beliebigen Punktes einer Arterie in Bezug auf einen interessierenden Bereich des Objekts und Erhalten einer ersten Zeit-Intensitäts-Kurve für ein erstes Voxel in dem MRA-Bild, das dem ausgewählten Punkt entspricht (S11); und
Auswählen eines beliebigen Punktes einer Vene, die mit dem interessierenden Bereich des Objekts in Beziehung steht, und Erhalten einer zweiten Zeitintensitätskurve für ein zweites Voxel in dem MRA, das dem ausgewählten Punkt entspricht (S12);
Bestimmen von drei Zielbildrahmen aus der Vielzahl von 3-dimensionalen Bildrahmen (S20), wobei die drei Zielbildrahmen Folgendes umfassen
einen ersten Zielbildrahmen, der einem Arterienspitzenzeitpunkt entspricht,
einen zweiten Zielbildrahmen, der einem Venen-Spitzenzeitpunkt entspricht, und
einen dritten Zielbildrahmen, der einem Zwischenzeitpunkt entspricht, der zwischen dem Arterienspitzenzeitpunkt und dem Venenspitzenzeitpunkt liegt, wobei
der erste Zielbildrahmen auf der Grundlage der ersten Zeitintensitätskurve bestimmt wird, wobei der erste Zielbildrahmen dem Arterienspitzenzeitpunkt entspricht, der eine maximale Intensität in der ersten Zeitintensitätskurve darstellt,
der zweite Zielbildrahmen auf der Grundlage der zweiten Zeitintensitätskurve bestimmt wird, wobei der zweite Zielbildrahmen dem Venen-Spitzenzeitpunkt entspricht, der eine maximale Intensität in der zweiten Zeitintensitätskurve darstellt, und
der dritte Zielbildrahmen auf der Grundlage der ersten Zeitintensitätskurve und der zweiten Zeitintensitätskurve bestimmt wird, wobei der dritte Zielbildrahmen einem Zwischenzeitpunkt zwischen dem Arterienspitzenzeitpunkt, der die maximale Intensität in der ersten Zeitintensitätskurve darstellt, und dem Venenspitzenzeitpunkt, der die maximale Intensität in der zweiten Zeitintensitätskurve darstellt, entspricht;
Bestimmen von drei Referenzbildrahmen, die dem ersten, zweiten und dritten Zielbildrahmen entsprechen, aus den Bildrahmen (S30), wobei jeder der verbleibenden Bildrahmen außer dem ersten, zweiten und dritten Zielbildrahmen als Referenzbildrahmen bestimmt wird;
wobei ein Bildrahmen, der eine minimale Intensität in der ersten Zeitintensitätskurve repräsentiert, als der Referenzbildrahmen für den ersten Zielbildrahmen bestimmt wird; oder
wobei ein Bildrahmen, der eine minimale Intensität in der zweiten Zeitintensitätskurve repräsentiert, als der Referenzbildrahmen für den zweiten Zielbildrahmen bestimmt wird;
Erhalten von drei Teilen von Subtraktionsdaten durch Subtrahieren von Daten jedes Referenzbildrahmens von Daten der entsprechenden drei Zielbildrahmen (S40); und
Erhalten des mindestens einen Querschnittsbildes für den interessierenden Bereich des Objekts auf der Grundlage der Subtraktionsdaten (S50).

2. Verfahren nach Anspruch 1, wobei
- - der Bildrahmen, der eine minimale Intensität in der ersten ZeitIntensitätskurve darstellt, oder
- der Bildrahmen, der eine minimale Intensität in der zweiten ZeitIntensitätskurve darstellt
als der dem dritten Zielbildrahmen entsprechende Referenzbildrahmen bestimmt wird.

3. Verfahren nach Anspruch 1, wobei der interessierende Bereich ein Gehirn des Objekts ist.

4. System zum Erhalten eines Querschnittsbildes zur Identifizierung von Perfusion unter Verwendung eines MRA-Bildes, wobei das System umfasst:
MRI-Ausrüstung, die so konfiguriert ist, dass sie das MRA-Bild für ein Objekt liefert, wobei das MRA-Bild
eine Vielzahl von 3-dimensionalen Bildrahmen enthält, die periodisch für eine bestimmte Zeitspanne aufgenommen wurden;
eine Benutzerschnittstelle (140), die so konfiguriert ist, dass sie die Auswahl eines beliebigen Punktes einer Arterie in Bezug auf einen interessierenden Bereich ermöglicht, und die so konfiguriert ist, dass sie die Auswahl eines beliebigen Punktes einer Vene in Bezug auf einen interessierenden Bereich ermöglicht;
einen Mikroprozessor, der konfiguriert ist,
um drei Zielbildrahmen aus der Vielzahl von 3-dimensionalen Bildrahmen zu bestimmen, wobei die drei Zielbildrahmen umfassen
einen ersten Zielbildrahmen, der einem Arterienspitzenzeitpunkt entspricht
einen zweiten Zielbildrahmen, der einem Venen-Spitzenzeitpunkt entspricht, und
einen dritten Zielbildrahmen, der einem Zwischenzeitpunkt zwischen dem Arterienspitzenzeitpunkt und dem Venenspitzenzeitpunkt entspricht,
um drei Referenzbildrahmen, die dem ersten, zweiten und dritten Zielbildrahmen entsprechen, aus den Bildrahmen zu bestimmen;
um drei Teile von Subtraktionsdaten durch Subtraktion der Daten jedes Referenzbildrahmens von Daten der entsprechenden drei Zielbildrahmen zu berechnen, und
um das mindestens eine Querschnittsbild für einen interessierenden Bereich des Objekts auf der Grundlage der Subtraktionsdaten zu erhalten,
wobei der Mikroprozessor konfiguriert ist,
um eine erste Zeitintensitätskurve für ein erstes Voxel in dem MRA-Bild zu berechnen, das einem beliebigen Punkt einer Arterie entspricht, die mit dem interessierenden Bereich in Beziehung steht, der von einem Benutzer mit der Benutzerschnittstelle (140) ausgewählt wurde; und
um eine zweite Zeit-Intensitäts-Kurve für ein zweites Voxel in dem MRA-Bild zu berechnen, das einem beliebigen Punkt einer Vene entspricht, die mit dem interessierenden Bereich in Beziehung steht, der von einem Benutzer mit der Benutzungsschnittstelle (140) ausgewählt wurde;
um den ersten Zielbildrahmen basierend auf der ersten Zeitintensitätskurve zu bestimmen, wobei der erste Zielbildrahmen dem Arterienspitzenzeitpunkt entspricht, der eine maximale Intensität in der ersten Zeitintensitätskurve darstellt,
um den zweiten Zielbildrahmen auf der Grundlage der zweiten Zeitintensitätskurve zu bestimmen, wobei der zweite Zielbildrahmen dem VenenSpitzenzeitpunkt entspricht, der eine maximale Intensität in der zweiten Zeitintensitätskurve darstellt, und
um den dritten Zielbildrahmen auf der Grundlage der ersten und zweiten Zeitintensitätskurve zu bestimmen, wobei der dritte Zielbildrahmen einem Zwischenzeitpunkt zwischen dem Arterienspitzenzeitpunkt, der die maximale Intensität in der ersten Zeitintensitätskurve darstellt, und dem Venenspitzenzeitpunkt, der die maximale Intensität in der zweiten Zeitintensitätskurve darstellt, entspricht; und
wobei der Mikroprozessor einen beliebigen der verbleibenden Bildrahmen mit Ausnahme des ersten, zweiten und dritten Zielbildrahmens als den Referenzbildrahmen bestimmt, und wobei der Mikroprozessor konfiguriert ist, um Folgendes zu bestimmen
einen Bildrahmen, der eine minimale Intensität in der ersten Zeit-Intensitäts-Kurve darstellt, als den Referenzbildrahmen für den erste Zielbildrahmen, oder
einen Bildrahmen, der eine minimale Intensität in der zweiten Zeitintensitätskurve darstellt, als den Referenzbildrahmen für das zweite Zielbild.

5. Das System nach Anspruch 4, das ferner Folgendes umfasst eine Anzeige, die so konfiguriert ist, dass sie das mindestens eine vom Mikroprozessor erhaltene Querschnittsbild anzeigt

6. System nach Anspruch 4, wobei der Mikroprozessor so konfiguriert ist, dass er Folgendes bestimmt
- den Bildrahmen, der eine minimale Intensität in der ersten ZeitIntensitätskurve darstellt, oder
- den Bildrahmen, der eine minimale Intensität in der zweiten ZeitIntensitätskurve darstellt
als der dem dritten Zielbildrahmen entsprechende Referenzbildrahmen.

## Revendications

1. - Procédé d'obtention d'une image en coupe transversale pour identifier une perfusion à l'aide d'une image d'ARM, le procédé comprenant :
obtenir une image d'ARM comprenant une pluralité de trames d'images tridimensionnelles qui ont été périodiquement capturées pendant une période de temps spécifique pour un objet à l'aide d'un équipement d'IRM (S10), l'image d'ARM étant obtenue par le biais d'un schéma d'injection de contraste ;
sélectionner un point arbitraire d'une artère associée à une zone d'intérêt de l'objet et obtenir une première courbe temps-intensité pour un premier voxel dans l'image d'ARM correspondant au point sélectionné (S11) ; et
sélectionner un point arbitraire d'une veine associée à la zone d'intérêt de l'objet et obtenir une seconde courbe temps-intensité pour un second voxel dans l'ARM correspondant au point sélectionné (S12) ;
déterminer trois trames d'images cibles à partir de la pluralité de trames d'images tridimensionnelles (S20), les trois trames d'images cibles comprenant
une première trame d'image cible correspondant à un point temporel de crête d'artère,
une deuxième trame d'image cible correspondant à un point temporel de crête de veine, et
une troisième trame d'image cible correspondant à un point temporel intermédiaire qui est entre le point temporel de crête d'artère et le point temporel de crête de veine,
dans lequel
la première trame d'image cible est déterminée sur la base de la première courbe temps-intensité, la première trame d'image cible correspondant au point temporel de crête d'artère qui représente une intensité maximale dans la première courbe temps-intensité,
la deuxième trame d'image cible est déterminée sur la base de la seconde courbe temps-intensité, la deuxième trame d'image cible correspondant au point temporel de crête de veine qui représente une intensité maximale dans la seconde courbe temps-intensité, et
la troisième trame d'image cible est déterminée sur la base de la première courbe temps-intensité et de la seconde courbe temps-intensité, la troisième trame d'image cible correspondant à un point temporel intermédiaire entre le point temporel de crête d'artère qui représente l'intensité maximale dans la première courbe temps-intensité et le point temporel de crête de veine qui représente l'intensité maximale dans la seconde courbe temps-intensité ;
déterminer trois trames d'image de référence correspondant à la première, à la deuxième et à la troisième trame d'image cible à partir des trames d'image (S30), n'importe laquelle des trames d'image restantes, à l'exception de la première, de la deuxième et de la troisième trame d'image cible, étant déterminée en tant que trames d'image de référence ;
dans lequel une trame d'image qui représente une intensité minimale dans la première courbe temps-intensité est déterminée en tant que trame d'image de référence pour la première trame d'image cible ; ou
dans lequel une trame d'image qui représente une intensité minimale dans la seconde courbe temps-intensité est déterminée en tant que trame d'image de référence pour la deuxième trame d'image cible ;
obtenir trois éléments de données de soustraction en soustrayant des données de chaque trame d'image de référence à des données des trois trames d'image cibles correspondantes (S40) ; et
obtenir l'au moins une image en coupe transversale pour la zone d'intérêt de l'objet sur la base des données de soustraction (S50).

2. - Procédé selon la revendication 1, dans lequel
- la trame d'image qui représente une intensité minimale dans la première courbe temps-intensité, ou
- la trame d'image qui représente une intensité minimale dans la seconde courbe temps-intensité
est déterminée en tant que trame d'image de référence correspondant à la troisième trame d'image cible.

3. - Procédé selon la revendication 1, dans lequel la zone d'intérêt est un cerveau de l'objet.

4. - Système d'obtention d'une image en coupe transversale pour identifier une perfusion à l'aide d'une image d'ARM, le système comprenant :
un équipement d'IRM configuré pour fournir l'image d'ARM pour un objet, l'image d'ARM comprenant une pluralité de trames d'image tridimensionnelles qui ont été capturées périodiquement pendant une période de temps spécifique ;
une interface utilisateur (140) configurée pour permettre la sélection d'un point arbitraire d'une artère associée à une zone d'intérêt, et configurée pour permettre la sélection d'un point arbitraire d'une veine associée à une zone d'intérêt ;
un microprocesseur configuré pour
déterminer trois trames d'image cibles à partir de la pluralité de trames d'image tridimensionnelles, les trois trames d'image cibles comprenant
une première trame d'image cible correspondant à un point temporel de crête d'artère,
une deuxième trame d'image cible correspondant à un point temporel de crête de veine, et
une troisième trame d'image cible correspondant à un point temporel intermédiaire entre le point temporel de crête d'artère et le point temporel de crête de veine,
déterminer trois trames d'image de référence correspondant à la première, à la deuxième et à la troisième trame d'image cible à partir des trames d'image ;
calculer trois éléments de données de soustraction en soustrayant des données de chaque trame d'image de référence à des données des trois trames d'image cibles correspondantes, et
obtenir l'au moins une image en coupe transversale pour une zone d'intérêt de l'objet sur la base des données de soustraction,
dans lequel le microprocesseur est configuré pour
calculer une première courbe temps-intensité pour un premier voxel dans l'image d'ARM correspondant à un point arbitraire d'une artère associée à la zone d'intérêt sélectionné par un utilisateur à l'aide de l'interface utilisateur (140) ; et
calculer une seconde courbe temps-intensité pour un second voxel dans l'image d'ARM correspondant à un point arbitraire d'une veine associée à la zone d'intérêt sélectionné par un utilisateur à l'aide de l'interface utilisateur (140) ;
déterminer la première trame d'image cible sur la base de la première courbe temps-intensité, la première trame d'image cible correspondant au point temporel de crête d'artère qui représente une intensité maximale dans la première courbe temps-intensité,
déterminer la deuxième trame d'image cible sur la base de la seconde courbe temps-intensité, la deuxième trame d'image cible correspondant au point temporel de crête de veine qui représente une intensité maximale dans la seconde courbe temps-intensité, et
déterminer la troisième trame d'image cible sur la base des première et seconde courbes temps-intensité, la troisième trame d'image cible correspondant à un point intermédiaire entre le point temporel de crête d'artère qui représente l'intensité maximale dans la première courbe temps-intensité et le point temporel de crête de veine qui représente l'intensité maximale dans la seconde courbe temps-intensité ; et
dans lequel le microprocesseur détermine n'importe laquelle des trames d'image restantes, à l'exception des première, deuxième et troisième trames d'image cibles, en tant que trame d'image de référence, et dans lequel le microprocesseur est configuré pour déterminer
une trame d'image qui représente une intensité minimale dans la première courbe temps-intensité en tant que trame d'image de référence pour la première trame d'image cible, ou
une trame d'image qui représente une intensité minimale dans la seconde courbe temps-intensité en tant que trame d'image de référence pour la deuxième trame d'image cible.

5. - Système selon la revendication 4, comprenant en outre :
un dispositif d'affichage configuré pour afficher l'au moins une image en coupe transversale obtenue par le microprocesseur.

6. - Système selon la revendication 4, dans lequel le microprocesseur est configuré pour déterminer
- la trame d'image qui représente une intensité minimale dans la première courbe temps-intensité, ou
- la trame d'image qui représente une intensité minimale dans la seconde courbe temps-intensité
en tant que trame d'image de référence correspondant à la troisième trame d'image cible.
